# EUROPEAN PATENT SPECIFICATION

(11) **EP 2 961 429 B1**
(45) Date of publication and mention of the grant of the patent: **27.12.2017**
(21) Application number: 14716005.5
(22) Date of filing: 28.02.2014
(51) Int. Cl.: A61K 41/00, A61K 49/18, B01J 2/00, A61N 1/40, A61N 2/02, A61P 35/00, A61B 5/055, A61K 9/51, G01R 33/28

(54) **METHOD FOR THE PRODUCTION OF SUB-MICROMETRIC PARTICLES AND THEIR THERANOSTIC USE IN ONCOLOGY WITH A SPECIFIC APPARATUS**
VERFAHREN ZUR HERSTELLUNG VON SUBMIKROMETRISCHEN PARTIKELN UND THERANOSTISCHE VERWENDUNG DAVON IN DER ONKOLOGIE MITTELS EINER SPEZIFISCHEN VORRICHTUNG
MÉTHODE DE PRODUCTION DE PARTICULES SUBMICROMÉTRIQUES ET LEUR UTILISATION THÉRANOSTIQUE EN ONCOLOGIE À L'AIDE D'UN APPAREIL SPÉCIFIQUE

(30) Priority: 28.02.2013 IT TV20130027
(43) Date of publication of application: 06.01.2016
(73) Proprietor: MBN Nanomaterialia SpA, 31050 Vascon di Carbonera (IT)
(72) Inventor: MATTEAZZI, Paolo, I-31100 Treviso (IT); GHERLINZONI, Filippo, I-31100 Treviso (IT); GOTTARDI, Michele, I-31029 Vittorio Veneto (Treviso) (IT)
(74) Representative: Dragotti, Gianfranco
(86) International application number: PCT/IB2014/059337
(87) International publication number: WO 2014/132235

(56) References cited:
- EP-A1- 2 283 895
- WO-A1-97/07917
- WO-A1-2012/085782
- WO-A1-2013/019137
- NARUSE S. ETA L.: "Radiofrequency hyperthermia with successive monitoring of its effects on tumors using NMR spectroscopy", PROC. NATL. ACAD.SCI. USA, vol. 83, 2 November 1986 (1986-11-02), pages 8343-8347, XP002714488,
- OREL V E ET AL: "Analysis of the influence of a mechano- and magnetochemically synthesized magnetosensitive complex on the basis of nanoparticles of Fe3O4, doxorubicin, and electromagnetic irradiation on animals with Guerin carcinoma", DOPOVIDI NACIONALNOI AKADEMII NAUK UKRAINI, NAUKOVA DUMKA, KIEV, UK , no. 8 1 January 2010 (2010-01-01), pages 208-211, XP008165016, ISSN: 1025-6415 Retrieved from the Internet: URL:http://archive.nbuv.gov.ua/portal/all/ reports/2010-08/10-08-33.pdf
- JUN LU ET AL: "Solid-state synthesis of monocrystalline iron oxide nanoparticle based ferrofluid suitable for magnetic resonance imaging contrast application", NANOTECHNOLOGY, IOP, BRISTOL, GB, vol. 17, no. 23, 14 December 2006 (2006-12-14), pages 5812-5820, XP020104333, ISSN: 0957-4484, DOI: 10.1088/0957-4484/17/23/017
- LOMAEVA S. F. ET AL.: "The formation of the structural and phase composition and magnetic properties of Fe(Fe3C, FeSiC)-SiO2 nanocomposites upon mechnochemical alloying", THE PHYSICS OF METALS AND METALLOGRAPHY, vol. 109, 5 May 2010 (2010-05-05), pages 534-546, XP008165005,
- PERSCHINA A. G. ET AL.: "Adsorption of proteins on particles of nanosized powder of CoFe2O4", NANOSYSTEM, NANOMATERIALS, NANOTECHNOLOGY, vol. 6, 1 March 2008 (2008-03-01), pages 1019-1028, XP008165017,
- OREL V E ET AL: "Mechanochemically activated doxorubicin nanoaparticles in combination with 40 MHz frequency irradiation on A-549 lung carcinoma cells", DRUG DELIVERY, ACADEMIC PRESS, ORLANDO, FL, US, vol. 12, no. 3, 1 January 2005 (2005-01-01), pages 171-178, XP008165249, ISSN: 1071-7544, DOI: 10.1080/10717540590932007
- Jonathan Gunn ET AL: "A Simple and Highly Sensitive Method for Magnetic Nanoparticle Quantitation Using 1H-NMR Spectroscopy", Biophysical Journal, vol. 97, no. 9, 1 November 2009 (2009-11-01), pages 2640-2647, XP55295280, US ISSN: 0006-3495, DOI: 10.1016/j.bpj.2009.08.013

## Description

### Field of the invention and explanatory preamble

The present invention relates to a method for the production of particles of sub-micrometric size and to theranostic use thereof in oncology, i.e. a use in which the detection of tumours, in particular of malignant neoplasms, and the subsequent medical treatment thereof are closely associated.

As will be seen, the particles according to the present invention comprise nanocrystals, i.e., as defined by current scientific literature, crystallographically ordered agglomerations of a substance with characteristic dimensions in the range typically of less than 100 nm. Such an ordered agglomeration is separated from other nanocrystals by grain boundaries namely may consist of a single particle. The nanocrystals may also take the form of clusters of a certain number of nanocrystals so as to form larger size particles.

### State of the art

The most widespread therapeutic method used for malignant neoplasms is chemotherapy in which drugs are employed. However, in the case of those types of neoplasms localized in so-called "sanctuary sites" such as the central nervous system (CNS) and the testicle, the penetration of anti-neoplastic drugs is hindered by the presence of the blood-brain barrier or blood-testicle barrier (referred to henceforth herein as BBB and BTB, respectively).

The strategy used nowadays to overcome the obstacle posed by the BBB and BTB essentially consists in using supramaximal doses of said drugs, but this gives rise to a high risk of immediate side effects in the patients, such as a severe and prolonged reduction of the medullar function (with a consequent high risk of haemorrhage or infection), or delayed side effects, such as cognitive, cardiac or gonadal dysfunctions with reduced fertility.

Radiotherapy, namely the use of ionizing radiation, is another well-established method, which is frequently used in synergy with chemotherapy, to induce the necrosis of neoplastic masses. However, a part of the healthy tissues of the patient - which is albeit increasingly smaller with improvements to the method and the equipment for implementing it - is exposed to the radiation, with the well-known side effects.

In oncology for therapeutic purposes, during the last few years, considerable attention has been focussed on a method called magnetic hyperthermia based on obtaining the localized heating of the tumour cells which in particular are less resistant to heat than healthy cells. The method envisages the introduction, generally into the tumour cells themselves, of particles consisting at least partly of materials suitable for being subsequently heated inductively by means of application of an external electromagnetic field.

In this field a fundamental patent is US 4,106,488 which envisages administering to patients affected by malignant neoplasms, ferromagnetic, diamagnetic and paramagnetic particles for which tumour cells have a greater affinity than healthy cells. The magnetic particles, which are encapsulated in materials able to be removed after a predetermined time and kept suspended in suitable physiological solutions, are introduced *in vivo* and conveyed towards the neoplasms since they have been previously combined with radioisotopes. At the end of a 12-hour period following administration, the application of an external electromagnetic field of suitable intensity results in overheating of the magnetic particles as a result of the inductive currents, which causes melting of the encapsulation materials and raises the temperature of the tumour cells to values such that they are destroyed.

In addition to the drawbacks due to the use of radioisotopes and the need for disposal of the encapsulating materials from the patient's body, it is necessary to mention also the amount of time which lapses between administration of the magnetic particles and heating thereof, namely when the tumour cells are actually destroyed. In any case it should be noted that this patent does not provide teachings relating to the methods for the production and selection of the magnetic particles, which are instead discussed in more recent documents.

Among the latter, the patent US 5,441,746 describes a complex method for producing particles which have a magnetic, for example ferrite, core which initially comprises the addition in the liquid state of alkalis to bivalent and trivalent metallic hydroxides, followed by a mixing step for obtaining amorphous metallic gels which have a diameter of the order of 100 nm and finally a heating step performed in the presence of oxygen in order to dehydrate the gel and control the dimensions of the resultant crystalline metal oxides. In addition to the magnetic core, the particles comprise a dual coating which not only increases the absorption of the external electromagnetic waves by the core, and therefore overheating thereof, but also allows the particles to pass through specific physiological membranes which in the body protect certain types of cells.

The patent US 7,122,030 refers to a therapeutic method for the treatment of malignant neoplasms by means of hyperthermia where ferroelectric particles are used, namely crystalline dielectric particles which may receive a permanent electrical polarization from a variable electric field. The ferroelectric particles are provided with a coating layer, for example a biocompatible and biodegradable polymer, so that they assume dimensions also of a significant nature (up to 15 µm) and are able to reach tumour cells situated also deep within the patient's body. The method is carried out under the guidance of a control system which comprises the measurement of the temperature of the particles and the parameters of the variable electric field so that the tumour cells reach a temperature (with a variation range of between 41.5 and 50°C) which results in their destruction, while the healthy cells are not damaged. The patent does not provide any teachings as to the method for producing the ferroelectric particles, in particular their coating.

In addition to the above observations in connection with individual patents, the following may be deduced from examination of a broad and varied cross-section of the prior art on the subject of magnetic hyperthermia:
a) the methods for obtaining particles which are ready for use are most likely unsuitable for production on an industrial scale since they are particularly complex and/or involve particularly long process times: for example, in order to obtain the magnetic cores alone, without considering the subsequent steps where they are coated and/or functionalized, production steps such as synthesis by means of thermal decomposition of organo-metallic compounds in high-boiling organic solvents and methods for hydrothermal synthesis with a duration of up to 72 hours have been proposed;
b) the overall execution of the method for therapeutic purposes envisages that the patient must undergo numerous steps (administration of the particles, viewing of their arrival in the neoplasms to be treated, heating induction of the magnetic particles) which are performed in different locations and at different moments in time, with an increase in the costs for the health cares structure and greater demands, if not inconvenience, for the patient.

The prior art includes also the following documents, the author of which is one of the present inventors and the contents of which are cited herein integrally as a reference source: P. MATTEAZZI, Reduction of haematite with carbon by room temperature ball milling, in Materials Science and Engineering, A149 (1991), which demonstrates the feasibility of magnetic nanocrystals in a high-energy mill and the patent application WO2012/085782 relating to a mechanochemical reactor with a high performance also from a technical/economic point of view. OREL V E ET AL, DOPOVIDI NACIONALNOI AKADEMII NAUK UKRAINI no. 8 1 January 2010 (2010-01-01), pages 208-211, discloses the analysis of the influence of a mechano- and magnetochemically synthesized magnetosensitive complex on the basis of nanoparticles of Fe3O4, doxorubicin, and electromagnetic irradiation on animals with Guerin carcinoma. It is pointed out that, in accordance with the scientific literature (for example the aforementioned article), "mechanochemical treatment" is understood as meaning the transformations produced by the impact of masses (milling bodies) which have a high kinetic energy per unit of homogeneous or heterogeneous material (volume, weight) exposed to impact. These mechanochemical transformations involve effects such as variations in the mixing state, structure and size of the crystals, distribution of the phases, state of aggregation or state of chemical combination of the elements and compounds present

### Object of the invention

The object of the present invention therefore is to overcome the shortcomings and/or drawbacks of that which has been disclosed hitherto with regard to magnetic hyperthermia by proposing a therapeutic method which allows the treatment of all types of malignant neoplasm, even neoplasms localized in "sanctuary sites" such as the central nervous system and testicle, and which is able to replace chemotherapy and other treatment methods established in oncology, or at least reduce the use thereof, essentially by:
- using sub-micrometric particles produced using methods and apparatus of proven technological and economic efficiency and using substances which, at least in preferred embodiments of the invention, are commonly available;
- performing theranostics of the malignant neoplasms by means of a material and temporal combination of diagnosis and therapy using an apparatus, which is novel but based on already well-known systems, allowing the reduction of investment and operating costs affecting health care structures as well as the demands and inconveniences for patients.

### Subject-matter of the invention

In order to achieve this object and other objects, a first subject of the present invention consists in a method for producing particles with sub-micrometric dimensions for *in vivo* oncological use according to claim 1, comprising the following steps:
A. mechanochemical treatment of homogeneous or heterogeneous, powdery, magnetic materials in a controlled atmosphere and, where appropriate, in the presence of a liquid phase of not more than 5% by volume, until a consistent mass comprising at least 80% by volume of magnetic nanocrystal aggregates is obtained. The aggregates have mainly dimensions smaller than 500 nm and are formed by nanocrystals mainly with dimensions smaller than 100 nm.
B. dispersion of said aggregates in a biocompatible fluid;
C. dimensional selection and distribution of the nanocrystal aggregates depending on their Curie temperature within a predefined variation range. (It should be remembered that the Curie temperature is the temperature for transition from the ferromagnetic state to the paramagnetic state and this temperatures depends on the dimensions of the crystal). It is carefully selected in order to limit the maximum heating temperature of the magnetic particles so as to cause destruction of the tumour cells, leaving the surrounding zones of healthy tissue undamaged;
D. functionalization of the magnetic nanocrystal aggregates obtained during step C so as to obtain a coating thereof which comprises molecules of one or more of the following types of substances: substances for which the tumour cells have a particular metabolic avidity; substances having a biochemical affinity with the tumour cells; substances having an affinity with the acid microenvironment which surrounds the neoplastic cells. In accordance with the Warburg effect (discovered in 1923 by O.H. Warburg, Nobel Prize winner in 1931), the tumour cells have a high avidity for monosaccharide carbohydrates such as glucose and produce an extra-cellular pH which is more acid than healthy tissues.

In a preferred embodiment, the mechanochemical treatment of step A is performed by loading the powdery materials into a high-energy mill or alternatively into a mechanochemical reactor such as that described in the patent application WO 2012/085782 which, as mentioned above, is herein integrally incorporated by reference.

In a further preferred embodiment of the present invention, in order to achieve functionalization for the metabolic avidity in step D, the aforementioned coating molecules are monosaccharide carbohydrates, preferably glucose, provided at a first end (distal end) with spacers having a substantially linear form. The second end (proximal end) of the spacers is provided with a group having a high affinity towards the nanocrystals (for example chosen from among carboxylates, phosphonates or phosphates). In other words, the aforementioned molecules, for example glucose, form the outermost part of the coating of the magnetic nanocrystal aggregates.

A second subject of the present invention consists in the sub-micrometric particles produced using this method and able to be employed in the treatment of any type of malignant neoplasm, including neoplasms localized in "sanctuary sites" such as the central nervous system and the testicle.

A third subject of the present disclosure is an *in vivo* oncological use of the said sub-micrometric particles; this type of use is nowadays commonly referred to as being theranostic since, during it, the diagnosis (and hence also monitoring) and the therapy of the malignant neoplasms are performed immediately one after another, i.e. without interruption. The said use is performed in accordance with the following sequence of steps:
A. by means of a biologically compatible solution the introduction, intravenously, into the patient's body, of sub-micrometric particles comprising magnetic nanocrystal aggregates functionalized with a suitable coating. The nanocrystals are selected so that their Curie temperature is within a predetermined variation range. The coating of the sub-micrometric particles comprises molecules of one or more of the following types of substances: substances for which the tumour cells have a particular metabolic avidity; substances having a biochemical affinity with the said cells; substances having a chemical affinity with the prevalent acidity of the tumoural microenvironment;
B. the localization of the tumour cells by means of a magnetic resonance imaging (MRI) system which uses the tracing effect of the magnetic nanocrystals. In fact, the latter, owing to mutual attraction between tumour cells (and the tumoural microenvironment) and the coating molecules of the sub-micrometric particles, agglomerate (i.e. are concentrated in a substantially significant manner) in the tumoural tissues namely not only inside the tumour cells, but also in contact with them and in any case in the immediate vicinity thereof, for example in the region of the BBB and the BTB. Therefore, during this step B, owing to the present invention, the neoplasms may be diagnosed and monitored with extreme precision;
C. the application to the patient's body, immediately following, i.e. without interruption after the preceding step B, of an external, variable, electromagnetic field with a predetermined intensity and frequency. The field is located on the tumoural masses defined in step B. According to a preferred embodiment of the present invention, the electromagnetic field is generated by means known per se which are added to a machine, also known per se, of the magnetic resonance imaging (MRI) system, with which the preceding step B of this method is performed;
D. the electromagnetic field generates in the magnetic nanocrystals the inductive currents which cause overheating thereof and consequently heating of the magnetic nanocrystals concentrated in the region of the tumour cells with temperatures limited by the Curie temperature range. According to the present invention, this step is performed in a very precise manner by means of detection of the attenuation of the response which is provided by the tracer and which corresponds to the fact that the magnetic nanocrystals reach the Curie temperatures with the consequent necrosis of the tumour cells. As soon as the temperature of the magnetic nanocrystals exceeds the maximum value (Tₘₐₓ) of the predefined variation range of the Curie temperature, the generation of the electromagnetic field is interrupted, preventing damage to the surrounding healthy tissue in which, as already mentioned, the magnetic nanocrystals are present in a significantly smaller concentration.

A last subject of the present invention is an apparatus suitable for the use described above, for implementing this method.

The above reveals two kinds of advantages offered by the present invention which clearly provides an effective theranostic approach. The first advantage is that the posture of the patient remains unchanged from start to finish, with obvious less inconvenience. The second which favours the health care establishment where this approach is adopted, consists in: lower investment costs for the machinery used in the oncology department, together with a smaller space occupied; reduction in the amount of time spent by the patient in the health care structure.

### Embodiments of the invention

Hereinbelow a number of preferred embodiments are described in detail in order to highlight better the characteristic features and advantages of the present invention compared to the prior art. It is understood, however, that other embodiments thereof may be provided and realized and/or variants thereof may be developed within the scope of protection defined in the claims below.

Firstly the three main steps of the method for the production of sub-micrometric particles for oncological use are described below with the aid of a number of examples.

### Step A - Mechanochemical treatment of powdery materials

According to experiments carried out on the second embodiment of the mechanochemical reactor described in the already mentioned patent application. WO 2012/085782 the feasibility of the following treatments has been demonstrated.

### Direct conversion

Magnetic materials consisting of oxide powders (e.g. iron oxides), or carbides (e.g. iron carbides), intermetallic compounds or other compounds or magnetic alloys are treated, namely ground, in the solid state in a neutral atmosphere and without addition of heat. The duration of the treatment is the time needed to obtain a mass which consists principally (at least 80% by volume) of aggregates with dimensions smaller than 500 nm, the aggregates being in turn formed by magnetic nanocrystals, the dimensions of which are smaller than 100 nm and typically included within the range of 10-50 nm. In certain circumstances, in order to obtain the desired result, it may be required to introduce into the mill also suitable process agents (such as alcohols, stearates, hydrocarbons, water, although any liquid phase present during treatment is not greater than 5% by volume) or integrate the milled product into the reactor with a conventional disintegration treatment by means of ultrasound or the like.

### Mechanochemical synthesis

Iron in powder form (pure element) is treated, namely ground, in the solid state and without addition of heat inside a mechanochemical reactor where an atmosphere with an adjustable oxygen content is maintained for the time needed to obtain the reaction Fe + O → FeOₓ, namely to obtain bivalent or trivalent magnetic oxides depending on the oxygen content inside the reactor. The duration of the treatment is the time needed to obtain a mass which consists principally (at least 80% by volume) of aggregates with dimensions smaller than 500 nm, the aggregates being in turn formed by magnetic nanocrystals, the dimensions of which are smaller than 100 nm and typically within the range of 10-50 nm. In the same way a large variety of compounds and magnetic alloys of iron or other metals may be produced.

### Mechanochemical synthesis and transformation

Powdery magnetic materials consisting of oxides (e.g. iron oxides) or carbides (e.g. iron carbides), intermetallic compounds or other compounds or metallic alloys may be obtained in forms, also of a mixed nature, with a combination of the treatments described above so as to obtain magnetic systems which may also be complex in nature. In a mechanochemical reactor transformation such as those indicated below by way of example are obtained:
Fe₂O₃ + Crₓ → Fe₂CrₓO₃ and Fe₂O₃ + Cr₂O₃ → FeₓCr_{y}O_{z} (mixed iron and chromium oxides)
Fe+C+Cr → FexCryCz (complex chromium carbide)
Co+C → CoCr (cobalt-chromium alloy).

In this case also the duration of the treatment is the time needed to obtain a mass which consists principally (at least 80% by volume) of aggregates with dimensions smaller than 500 nm, the aggregates being in turn formed by magnetic nanocrystals, the dimensions of which are mainly less then 100 nm and typically included within the range of 10-50 nm.

### Step C - Magnetic selection of the nanocrystals

For the purposes of the intended oncological use of the particles it is of fundamental importance to select a dimensional category of the nanocrystals corresponding to a correct Curie temperature, namely a temperature able to induce the necrosis of the tumour cells without damaging the healthy cells. For this purpose it is preferable that the Curie temperature of the magnetic nanocrystals should be in a range having a minimum value Tₘᵢₙ = 38°C and a maximum value Tₘₐₓ = 42°C.

The aggregates obtained for example in accordance with one of the aforementioned examples are dispersed in a liquid phase so as to obtain a dispersion of nanocrystal aggregates (Step B). Obviously, a biocompatible liquid phase, for example an aqueous solution, must be used, namely one such as not to cause any modification of the chemical composition of the nanocrystals and not alter their magnetic properties.

In order to select, for the purposes of the intended oncological use, all and only the magnetic nanocrystal aggregates which have a Curie temperature within the desired range, according to the present invention the procedure below is adopted. Firstly the solution is brought to the temperature Tₘₐₓ which is kept thermostatically controlled while, by means of a first magnetic filter immersed in the solution, all the magnetic nanocrystals which have a Curie temperature higher than the said value Tₘₐₓ are removed therefrom. The temperature of the solution is then lowered down to the temperature Tₘᵢₙ which is kept under thermostatic control, while, with the aid of a second magnetic filter, the nanocrystal aggregates with a Curie temperature within the range Tₘᵢₙ ÷ Tₘₐₓ are extracted for the subsequent oncological use.

### Step D - Functionalization of the nanocrystal aggregates

In the present invention, the primary aim of functionalization of the nanocrystal aggregates obtained and selected during the preceding steps is to produce a coating of the aggregates which maximizes the mutual attraction between tumoural masses *in vivo* and sub-micrometric particles.

According to the present invention, molecules of various types may be used in order to obtain the coating of the magnetic nanocrystal aggregates: substances for which the tumour cells have a particular metabolic avidity (for example monosaccharide carbohydrates, in particular glucose); substances having a biochemical affinity with the tumour cells (for example monoclonal anti-bodies); substances having an affinity with the acid microenvironment which surrounds the neoplastic cells (for example linear polysaccharides such as chitosan).

For example in the case of a coating with monosaccharide carbohydrates, the present invention envisages making use of standard organic synthesis procedures in order to form the biocompatible spacers with a linear form of varying length. A first end (distal end) of each spacer is provided with molecules, for example glucose, while the other end (proximal end) is provided with a group having a high affinity towards the magnetic properties of the nanocrystals (such as carboxylates, phosphonates and phosphates).

In the case of malignant neoplasms localized in so-called "sanctuary sites" such as the central nervous system (CNS) and the testicle, where the blood-brain barrier and the blood-testicle barrier are respectively present, the coating therefore has the effect of bringing the magnetic nanocrystals into direct contact with, even though not inside, the tumour cells. In other words, a concentration of the magnetic nanocrystals in the region around the tumour cells is obtained.

An example of implementation of the method illustrated above is provided below solely by way of a non-limiting example.

### Step A - Mechanochemical treatment

According to a possible embodiment of step A, a mechanochemical reactor of the type described in the already mentioned patent application WO2012/085782 provided with milling balls with a weight of 15 kg and operating with an oscillating frequency of 15 Hz, may be used. The powdery magnetic material introduced into the reactor for treatment may have an overall weight of 1.5 kg and may be magnetite (Fe3O4) with a mean distribution of the particles of 35µm: consequently the ratio between material to be treated and milling means is 1:10. After being subjected to a vacuum, the milling chamber of the reactor may be filled for example with 99.99% pure argon with 2% of added oxygen.

The treatment may last 4 hours and may give rise to about 1 kg of aggregates with dimensions of less than 400 nm. Then the aggregates may undergo characterization with X-ray diffraction and evaluation using the Scherrer equation. Advantageously, the aggregates may be formed by magnetite crystals with an average size smaller than 10 nm (magnetic nanocrystals).

### Step B - Dispersion (disaggregation) of the aggregates

For this step a conventional grinding mill for example of the type commercially distributed by Union Process Inc. (USA) and provided with milling balls made of zirconium oxide with a diameter of 300µ and overall weight of 2 kg, filled with 2 litres of water and operating at a speed of rotation of 100 rpm, may be used. 300g of powder (aggregates of magnetite nanocrystals obtained in Step A) may be loaded into the grinding mill. At the end of a process time of 2 hours an aqueous dispersion of particles may be obtained, the weight distribution thereof consisting 90% of particles with a size smaller than 60 nm which may be advantageously measured using a laser diffractometer, for example of the type commercially distributed by Malvern Instruments Ltd (UK). Using a set of filters with dimensions ranging from 8µm to 50nm it is possible to obtain a liquid which consists of about 2 litres of water and in which a solid dispersion of aggregate particles of magnetite nanocrystals with an average size of 10 nm for about 100 g is formed.

### Step C - Magnetic selection of the nanocrystals

The solution obtained at the end of Step B and temperature-controlled at 42°C may be passed through a first magnetic filter which is also kept at 42°C so as to capture the particles having a Curie temperature higher than this value. After lowering the temperature to 38°C, the solution thus selected may be passed through a second, different, magnetic filter kept at 38°C so as to capture the particles with a Curie temperature greater than 38°C and therefore inside the desired range, i.e. between 38°C (Tₘᵢₙ and 42°C (Tₘₐₓ for subsequent theranostic use. The "useful" particles of magnetite, namely those selected in this way, may total about 30 g in weight (10% compared to the initial 300 g).

The fluid selected with particles having a Curie temperature of less than 38°C may also be replenished, in the same weight proportion, with the selected particles in the temperature range 38-42°C, so as to form a system comprising the superparametric fraction at a body temperature (useful for basic imaging) supplemented with the particles useful for the treatment.

### Step D - Functionalization

In the present invention, the primary aim of functionalization of the nanocrystal aggregates obtained and selected during the preceding steps is to produce a coating of the aggregates which maximizes the mutual attraction between tumoural masses *in vivo* and sub-micrometric particles, which could be for example glucose. In the case of magnetic oxide particles, as in this example, it may be particularly useful, for example, to use a coating of the particles with silanes (silanization) which allows both the attachment of various carrier substances and the dispersion of the particles in water, facilitating their stability in an aqueous solution.

The particles selected in Step C may be dispersed in water in a concentration of 5% and the silanization may be performed using 3-aminopropyltriethoxysilane, leaving for example the dispersion at 40°C for 3 hours. By subsequently heating the same dispersion to 70°C with glucose in a concentrated aqueous solution, a fructosamine R-NH-CH2-CO-(CHOH)3-CH2OH which coats the single particle may be obtained according to the Amadori reaction. The stable dispersion thus obtained may be used directly for intravenous injection in the amount of 30 micromol Fe/kg of bodyweight.

According to the present invention, a preferred form of oncological use of the sub-micrometric particles is performed with a novel apparatus which is based on a conventional magnetic resonance imaging (MRI) system, substantially all the parts of which are maintained, and which further comprises one or more induction coils for generating an oscillating electromagnetic field, as well as means for regulating the parameters of the field and directing it towards the zone of the patient's body where the neoplasm is assumed to be present.

With this apparatus it is possible to perform both the diagnosis (and monitoring) as well as the treatment of the neoplasms, without interruption, i.e. in a continuous manner, while keeping the posture of the patient unchanged.

The diagnosis step is per se conventional in that it ascertains the location of the magnetic aggregates (present in the sub-micrometric particles previously introduced into the patient's body intravenously) in the region of the tumour cells, owing to the "attraction" which the latter exert on the molecules of their coating.

During therapy, the electromagnetic field generated by the induction coils results in heating of the nanocrystals which, following selection thereof, as already described above, remain with certainty within the predefined variation range of the Curie temperature. The heating is interrupted instantaneously while, at the same time, the nanocrystals are no longer "viewable" by the diagnostic means owing to the fact that they have lost their magnetic properties. As a result it is possible to establish a principle which ensures the specific and selective destruction of the tumour cells, without damaging the healthy tissues, including those which are in the immediate vicinity of the neoplasms.

More particularly, the theranostic method may be carried out for example on a visible mass traced in conventional MRI (magnetic resonance imaging). After prior treatment with cortisones and anti-histamines, the previously mentioned dispersion from step D selected within the Curie temperature range 38-43 °C may be injected intravenously. An induction coil with an induced field of 7 KA/m at the frequency of 100 KHZ may be used, for example, for the treatment, having been placed inside a magnetic resonance machine. The part to be treated is inserted inside the coil after anaesthesia. Before application of the field, the MRI causes a reduction in the signal in the region of the mass owing to the accumulation of the magnetic particles caused by the glucose carrier. Following application of the induced field, as described above, after about 5 minutes, the image in the region of the tumoural mass may be seen highlighted. This is due to the gradual entry into the super-paramagnetic field of the particles within the temperature range of 38-43 °C. The zones surrounding the tumoural mass, during the treatment which may last one hour in total, are stable in the MRI signal, with no substantial thermal alteration being detected therein. Advantageously, an analysis of the tumoural mass and the surrounding tissue may reveal a considerable degree of cellular necrosis of the tumoural mass without damage to the healthy tissue.

It is understood that other variations and embodiments are included within the scope of protection defined by the accompanying claims.

## Claims

1. Method for the production of sub-micrometric particles for the *in vivo* treatment of tumour cells, comprising the steps of:
A. mechanochemical treatment of homogeneous or heterogeneous, powdery, magnetic materials in a controlled atmosphere, and in the presence of a liquid phase of not more than 5% by volume, until a consistent mass with at least 80% by volume of magnetic aggregates mainly with dimensions smaller than 500 nm is obtained, said aggregates being formed by magnetic nanocrystals mainly with dimensions smaller than 100 nm;
B. dispersion of the magnetic aggregates in a fluid;
C. dimensional selection and distribution of the magnetic aggregates present in the said fluid depending on their Curie temperature within a predefined variation range(Tₘᵢₙ - Tₘₐₓ);
D. functionalization by coating the aggregates with substances designed to generate a mutual attraction between the tumour cells and the nanocrystals of the magnetic aggregates.

2. Method according to Claim 1, wherein the mechanochemical treatment takes place, in a high-energy mill or, alternatively, in a mechanochemical reactor, where an atmosphere with a controlled oxygen content is maintained.

3. Method according to Claim 1 or Claim 2, wherein the dimensional selection and distribution of the magnetic nanocrystal aggregates dispersed in a biocompatible fluid comprises in succession:
a) heating and keeping the fluid at the maximum temperature of the predefined variation range Tₘₐₓ;
b) removing from the fluid by means of a first magnetic filter the aggregates having a Curie temperature greater than Tₘₐₓ
c) cooling the fluid from which the said aggregates have been removed to the minimum temperature within the predefined variation range Tₘᵢₙ and keeping the fluid at said temperature;
d) removing from the fluid by means of a second magnetic filter the aggregates having a Curie temperature within the range Tₘᵢₙ - Tₘₐₓ with a view to subsequent oncological use.

4. Method according to any one of Claims 1 to 3, wherein functionalization of the aggregates is performed using molecules of substances for which the tumour cells have a particular metabolic avidity, such as monosaccharide carbohydrates, in particular glucose.

5. Method according to any one of Claims 1 to 3, wherein the functionalization of the aggregates is performed using molecules of substances having a biochemical affinity with the tumour cells such as monoclonal antibodies.

6. Method according to any one of Claims 1 to 3, wherein functionalization of the aggregates is performed using molecules of substances having an affinity with the acid microenvironment which surrounds the tumour cells such as linear polysaccharides, in particular chitosan.

7. Sub-micrometric particles for theranostic use in the treatment of neoplasms, obtained according to the methods of any one of the preceding claims, comprising:
• an aggregate of nanocrystals of at least one of the following magnetic materials: iron oxides, also of a mixed and complex nature; intermetallic compounds and iron-containing magnetic alloys; magnetic systems consisting of iron with other metals.
• a coating of the said aggregate of magnetic nanocrystals comprising molecules of at least one of the following types of substances: substances for which the tumour cells have a particular metabolic avidity; substances having a biochemical affinity with the tumour cells; substances having an affinity with the acid microenvironment which surrounds the neoplastic cells.

8. Sub-micrometric particles for theranostic use in oncology wherein said sub-micrometric particles are produced with the method according to any one of Claims 1 to 6 and/or Claim 7, comprising in succession and without interruption the steps of:
A. introducing intravenously the sub-micrometric particles into the patient's body using a compatible physiological solution;
B. diagnosis and/or monitoring of tumour cells by means of location of the magnetic nanocrystal aggregates concentrated in the vicinity or inside the said cells as a result of attraction of the molecules of the coating of the magnetic nanocrystal aggregates by means of a magnetic resonance imaging system known per se;
C. localized heating, inductively, of the magnetic nanocrystals to a temperature within a predefined variation range of the Curie temperature having, as an effect, the destruction of the tumour cells by means magnetic hyperthermia;
D. detection of the loss of the magnetic properties of the nanocrystals upon reaching the Curie temperature by means of the said imaging system known per se; and
E. simultaneous stoppage of said heating operation so as to ensure that the healthy tissue surrounding the tumour cells remains undamaged, since it is at a lower temperature.

9. Apparatus for implementing a theranostic use in oncology according to Claim 8, the apparatus comprising a magnetic resonance imaging (MRI) system known per se, sub-micrometric particles obtained with the method according to any one of claims 1-7, and also comprising means for generating an electromagnetic field designed to heat inductively, to a value within a predefined variation range of the Curie temperature, the magnetic nanocrystal aggregates forming part of the sub-micrometric particles previously introduced intravenously into the patient's body.

10. Apparatus for implementing a theranostic use in oncology according to Claim 9, **characterized in that** said heating means are designed to stop following the loss of the magnetic properties of the nanoparticles.

11. Apparatus according to either one of Claims 9 to 10, wherein the means for generating a variable electromagnetic field comprise one or more induction coils and means for varying the parameters of the said field and directing it towards the zone of the patient's body where a neoplasm is assumed to be present.

## Patentansprüche

1. Verfahren für die Herstellung von submikrometrischen Partikeln für die *in*-*vivo* Behandlung von Tumorzellen, aufweisend die Schritte:
A. mechanisch-chemische Behandlung von homogenen oder heterogenen, pulverförmigen, magnetischen Materialien in einer kontrollierten Atmosphäre und bei der Anwesenheit von einer flüssigen Phase von nicht mehr als 5 Volumen-% bis eine dickflüssige Masse mit mindestens 80 Volumen-% von magnetischen Zuschlagstoffen, hauptsächlich mit Abmessungen die kleiner als 500 nm sind, erhalten wird, wobei die Zugschlagstoffe durch magnetische Nanokristalle, hauptsächlich mit Abmessungen, die kleiner als 100 nm sind, gebildet werden;
B. Dispersion der magnetischen Zuschlagstoffe in einem Fluid;
C. maßliche Auslese und Verteilung der in dem Fluid vorhandenen magnetischen Zuschlagstoffe in Abhängigkeit von ihrer Curie-Temperatur innerhalb einer vordefinierten Streubreite (Tₘᵢₙ - Tₘₐₓ) ;
D. Funktionalisierung durch Beschichten der Zuschlagstoffe mit Substanzen, die konzipiert sind, eine gegenseitige Anziehung zwischen den Tumorzellen und den Nanokristallen der magnetischen Zuschlagstoffe zu erzeugen.

2. Verfahren gemäß Anspruch 1, wobei die mechanisch-chemische Behandlung in einer Hochleistungsmühle oder alternativ in einem mechanisch-chemischen Reaktor, in dem eine Atmosphäre mit einem kontrollierten Sauerstoffgehalt beibehalten wird, stattfindet.

3. Verfahren gemäß Anspruch 1 oder Anspruch 2, wobei die maßliche Auslese und Verteilung der in einem biokompatiblen Fluid dispergierten magnetischen Nanokristall-Zuschlagstoffe nacheinander aufweisen:
a) ein Heizen und ein Halten des Fluids bei der maximalen Temperatur der vordefinierten Streubreite Tₘₐₓ;
b) ein Entfernen der Zuschlagstoffe, die eine Curie-Temperatur, die größer als Tₘₐₓ ist, haben, von dem Fluid mittels eines ersten magnetischen Filters;
c) ein Kühlen des Fluids, von dem die Zuschlagstoffe entfernt wurden, auf die minimale Temperatur innerhalb der vordefinierten Streubreite Tₘᵢₙ und ein Halten des Fluids bei der Temperatur;
d) ein Entfernen der Zuschlagstoffe, die eine Curie-Temperatur innerhalb des Bereichs Tmin - Tmax haben, von dem Fluid mittels eines zweiten magnetischen Filters in Vorbereitung auf eine nachfolgende onkologische Verwendung.

4. Verfahren gemäß einem der Ansprüche 1 bis 3, wobei die Funktionalisierung der Zuschlagstoffe unter einer Verwendung von Molekülen von Substanzen, für die die Tumorzellen eine spezielle metabolische Avidität haben, wie etwa Monosaccharidkohlenhydrate, insbesondere Glukose, durchgeführt wird.

5. Verfahren gemäß einem der Ansprüche 1 bis 3, wobei die Funktionalisierung der Zuschlagstoffe unter einer Verwendung von Molekülen von Substanzen, die eine biochemische Affinität mit den Tumorzellen haben, wie etwa monoklonale Antikörper, durchgeführt wird.

6. Verfahren gemäß einem der Ansprüche 1 bis 3, wobei die Funktionalisierung der Zuschlagstoffe unter einer Verwendung von Molekülen von Substanzen, die eine Affinität mit dem sauren Mikroumfeld, das die Tumorzellen umgibt, haben, wie etwa lineare Polysaccharide, insbesondere Chitosan, durchgeführt wird.

7. Submikrometrische Partikel für eine theranostische Verwendung in der Behandlung von Neoplasmen, die gemäß den Verfahren von einem der vorangehenden Ansprüche erhalten werden, aufweisend:
• einen Zuschlagstoff aus Nanokristallen aus zumindest einem der folgenden magnetischen Materialien:
Eisenoxide, auch von einer gemischten und komplexen Beschaffenheit; intermetallische Zusammensetzungen und eisenhaltige magnetische Legierungen; aus Eisen mit anderen Metallen bestehende magnetische Systeme.
• eine Beschichtung des Zuschlagstoffs aus magnetischen Nanokristallen, die Moleküle von zumindest einem der folgenden Typen von Substanzen aufweist: Substanzen, für die die Tumorzellen eine spezielle metabolische Avidität haben; Substanzen, die eine biochemische Affinität mit den Tumorzellen haben; Substanzen, die eine Affinität mit dem sauren Mikroumfeld, das die neoplastischen Zellen umgibt, haben.

8. Submikrometrische Partikel, wobei die submikrometrischen Partikel mit dem Verfahren gemäß einem der Ansprüche 1 bis 6, und/oder gemäß Anspruch 7, hergestellt sind für eine theranostische onkologische Verwendung, die aufeinanderfolgend und ohne Unterbrechung die Schritte aufweist:
A. ein intravenöses Einbringen der submikrometrischen Partikel in den Körper eines Patienten unter Verwendung einer kompatiblen physiologischen Lösung;
B. ein Diagnostizieren und/oder ein Überwachen von Tumorzellen mittels eines Orts der magnetischen Nanokristall-Zuschlagstoffe, die als ein Ergebnis einer Anziehung der Moleküle der Beschichtung der magnetischen Nanokristall-Zuschlagstoffe in der Nähe oder innerhalb der Zellen konzentriert sind, durch einen per se bekannten Magnetresonanztomographen;
C. ein lokal begrenztes induktives Erhitzen der magnetischen Nanokristalle auf eine Temperatur innerhalb einer vordefinierten Streubreite der Curie-Temperatur, die die Zerstörung der Tumorzellen mittels magnetischer Überhitzung als einen Effekt hat;
D. ein Erfassen des Verlusts der magnetischen Eigenschaften der Nanokristalle beim Erreichen der Curie-Temperatur mittels des per se bekannten Bildgebungssystems;
und
E. ein gleichzeitiges Anhalten der Heizoperation, um sicherzustellen, dass das die Tumorzellen umgebende gesunde Gewebe ungeschädigt bleibt, da es bei einer niedrigeren Temperatur ist.

9. Einrichtung zum Ausführen einer theranostischen onkologischen Verwendung gemäß Anspruch 8, wobei die Einrichtung aufweist:
einen per se bekannten Magnetresonanztomographen (MRT),
mit dem Verfahren gemäß einem der Ansprüche 1 - 7 erhaltene submikrometrische Partikel, und
auch Mittel zum Erzeugen eines elektromagnetischen Felds, die konzipiert sind, induktiv auf einen Wert innerhalb einer vordefinierten Streubreite der Curie-Temperatur zu heizen, aufweisend, wobei die magnetischen Nanokristall-Zuschlagstoffe einen Teil der im Voraus intravenös in den Körper des Patienten eingeführten submikrometrischen Partikel bilden.

10. Einrichtung zum Ausführen einer theranostischen onkologischen Verwendung gemäß Anspruch 9, **dadurch gekennzeichnet, dass** die Heizmittel konzipiert sind, dem Verlust der magnetischen Eigenschaften der Nanopartikel folgend, anzuhalten.

11. Einrichtung gemäß einem der beiden Ansprüche 9 bis 10, wobei die Mittel zum Erzeugen eines variablen elektromagnetischen Felds eine oder mehrere Induktionsspulen und ein Mittel zum Verändern der Parameter des Felds und um es auf den Bereich des Körpers des Patienten zu richten, wo angenommen wird, dass sich ein Neoplasma befindet, aufweisen.

## Revendications

1. Procédé de production de particules sub-micrométriques pour le traitement *in vivo* de cellules tumorales, comprenant les étapes de :
A. traitement mécanochimique de matériaux magnétiques homogènes ou hétérogènes en poudre dans une atmosphère régulée, et en présence d'une phase liquide de pas plus de 5 % en volume, jusqu'à ce qu'une masse uniforme comportant au moins 80 % en volume d'agrégats magnétiques ayant principalement des dimensions inférieures à 500 nm soit obtenue, lesdits agrégats étant constitués de nanocristaux magnétiques ayant principalement des dimensions inférieures à 100 nm ;
B. dispersion des agrégats magnétiques dans un fluide ;
C. sélection dimensionnelle et répartition des agrégats magnétiques présents dans ledit fluide en fonction de leur température de Curie à l'intérieur d'une plage de variation prédéfinie (Tₘᵢₙ - Tₘₐₓ) ;
D. fonctionnalisation par revêtement des agrégats avec des substances conçues pour générer une attraction mutuelle entre les cellules tumorales et les nanocristaux des agrégats magnétiques.

2. Procédé selon la revendication 1, dans lequel le traitement mécanochimique a lieu, dans un broyeur à haute énergie ou, en variante, dans un réacteur mécanochimique, où une atmosphère ayant une teneur régulée en oxygène est maintenue.

3. Procédé selon la revendication 1 ou la revendication 2, dans lequel la sélection dimensionnelle et la répartition des agrégats de nanocristaux magnétiques dispersés dans un fluide biocompatible comprend successivement :
a) le chauffage et le maintien du fluide à la température maximale de la plage de variation prédéfinie Tₘₐₓ ;
b) l'extraction du fluide, à l'aide d'un premier filtre magnétique, des agrégats ayant une température de Curie supérieure à Tₘₐₓ
c) le refroidissement du fluide duquel lesdits agrégats ont été extraits à la température minimale à l'intérieur de la plage de variation prédéfinie Tₘᵢₙ et le maintien du fluide à ladite température ;
d) l'extraction du fluide, à l'aide d'un second filtre magnétique, des agrégats ayant une température de Curie à l'intérieur de la plage Tₘᵢₙ - Tₘₐₓ en vue d'une utilisation oncologique ultérieure.

4. Procédé selon l'une quelconque des revendications 1 à 3, dans lequel la fonctionnalisation des agrégats est réalisée en utilisant des molécules de substances pour lesquelles les cellules tumorales présentent une avidité métabolique particulière, telles que des glucides de type monosaccharide, en particulier du glucose.

5. Procédé selon l'une quelconque des revendications 1 à 3, dans lequel la fonctionnalisation des agrégats est réalisée en utilisant des molécules de substances présentant une affinité biochimique avec les cellules tumorales telles que des anticorps monoclonaux.

6. Procédé selon l'une quelconque des revendications 1 à 3, dans lequel la fonctionnalisation des agrégats est réalisée en utilisant des molécules de substances présentant une affinité avec le micro-environnement acide qui entoure les cellules tumorales telles que des polysaccharides linéaires, en particulier du chitosan.

7. Particules sub-micrométriques à usage théranostique dans le traitement des néoplasmes, obtenues selon les procédés de l'une quelconque des revendications précédentes, comprenant :
• un agrégat de nanocristaux d'au moins l'un des matériaux magnétiques suivants : oxydes de fer, également de nature mixte et complexe ; composés intermétalliques et alliages magnétiques contenant du fer ; systèmes magnétiques constitués de fer avec d'autres métaux.
• un revêtement dudit agrégat de nanocristaux magnétiques comprenant des molécules d'au moins l'un des types de substances suivants : substances pour lesquelles les cellules tumorales présentent une avidité métabolique particulière ; substances présentant une affinité biochimique avec les cellules tumorales ; substances présentant une affinité avec le micro-environnement acide qui entoure les cellules néoplasiques.

8. Particules sub-micrométriques à usage théranostique en oncologie dans lesquelles les particules sub-micrométriques sont produites à l'aide du procédé selon l'une quelconque des revendications 1 à 6 et/ou de la revendication 7, comprenant successivement et sans interruption les étapes de :
A. introduction par voie intraveineuse des particules sub-micrométriques dans le corps d'un patient à l'aide d'une solution physiologique compatible ;
B. diagnostic et/ou surveillance des cellules tumorales à l'aide de la localisation des agrégats de nanocristaux magnétiques concentrés à proximité ou à l'intérieur desdites cellules en résultat de l'attraction des molécules du revêtement des agrégats de nanocristaux magnétiques à l'aide d'un système d'imagerie par résonance magnétique connu en soi ;
C. chauffage localisé, par induction, des nanocristaux magnétiques à une température à l'intérieur d'une plage de variation prédéfinie de la température de Curie ayant, comme effet, la destruction des cellules tumorales par hyperthermie magnétique ;
D. détection de la perte des propriétés magnétiques des nanocristaux lorsqu'ils atteignent la température de Curie à l'aide dudit système d'imagerie connu en soi ; et
E. arrêt simultané de ladite opération de chauffage de manière à garantir que le tissu sain entourant les cellules tumorales reste intact, puisqu'il est à une température inférieure.

9. Appareil de mise en oeuvre d'une utilisation théranostique en oncologie selon la revendication 8, l'appareil comprenant
un système d'imagerie par résonance magnétique (IRM) connu en soi,
des particules sub-micrométriques obtenues par le procédé selon l'une quelconque des revendications 1 à 7, et
comprenant également un moyen de génération d'un champ électromagnétique conçu pour chauffer par induction, à une valeur à l'intérieur d'une plage de variation prédéfinie de la température de Curie, les agrégats de nanocristaux magnétiques faisant partie des particules sub-micrométriques précédemment introduites par voie intraveineuse dans le corps d'un patient.

10. Appareil de mise en oeuvre d'une utilisation théranostique en oncologie selon la revendication 9, **caractérisé en ce que** ledit moyen de chauffage est conçu pour s'arrêter après la perte des propriétés magnétiques des nanoparticules.

11. Appareil selon l'une ou l'autre des revendications 9 à 10, dans lequel le moyen de génération d'un champ électromagnétique variable comprend une ou plusieurs bobines d'induction et un moyen de variation des paramètres dudit champ et d'orientation de celui-ci vers la zone du corps du patient où un néoplasme est supposé être présent.
